# EUROPEAN PATENT APPLICATION

(11) **EP 4 063 361 A1**
(43) Date of publication of application: **28.09.2022**
(21) Application number: 20890684.2
(22) Date of filing: 18.11.2020
(51) Int. Cl.: C07D 413/14, A61K 31/5377, A61P 35/00

(54) **CRYSTAL FORMS OF FUSED RING COMPOUND, AND COMPOSITION THEREOF, PREPARATION METHOD THEREFOR AND APPLICATION THEREOF**

(30) Priority: 20.11.2019 CN 201911144271
(71) Applicant: Hangzhou Yusheng Medical Technology Co. Ltd, Hangzhou City, Zhejiang 311705 (CN)
(72) Inventor: HE, Qiaojun, Hangzhou City, Zhejiang 311705 (CN); CHEN, Binhui, Hangzhou City, Zhejiang 311705 (CN); ZHENG, Lin, Hangzhou City, Zhejiang 311705 (CN); WENG, Qinjie, Hangzhou City, Zhejiang 311705 (CN); YE, Ding, Hangzhou City, Zhejiang 311705 (CN); JIANG, Mingyong, Hangzhou City, Zhejiang 311705 (CN); GONG, Yi, Hangzhou City, Zhejiang 311705 (CN); WANG, Xiaoling, Hangzhou City, Zhejiang 311705 (CN)
(74) Representative: Dehns
(86) International application number: PCT/CN2020/129748
(87) International publication number: WO 2021/098716

(57) **Abstract**

Disclosed in the present disclosure are crystal forms of a fused ring compound, and a composition thereof, a preparation method therefor and use thereof. The crystal forms comprise a crystal form I, a crystal form II, a crystal form III, a crystal form IV and a crystal form V, which have, when using X-ray diffraction, characteristic diffraction peaks at about 11.3 degrees, 17.2 degrees and 21.1 degrees, at about 25.1 degrees, 21.2 degrees and 14.1 degrees, or at about 6.6 degrees, 13.4 degrees and 8.0 degrees, at about 11.8 degrees, 13.3 degrees and 16.7 degrees, and at about 6.5 degrees, 13.3 degrees and 20.0 degrees. The crystal form I or IV is obtained by dissolving a fused ring compound in a proper solvent, followed by stirring, filtering and drying. The use of crystal form I or crystal form IV in preparation of anti-cancer drugs for inhibiting phosphatidylinositol 3-kinase.The use thereof for targeted therapy for tumors, and for anti-inflammation or treatment of autoimmune diseases.

## Description

### Field of the Disclosure

The present disclosure specifically relates to a crystal form of a fused ring compound, a composition thereof, a preparation method thereof and uses thereof, which belongs to the art of medicinal chemistry.

### Background of the Disclosure

Malignant tumors are a group of major diseases that pose a serious threat to human health.Most of the small-molecule antitumor drugs as clinically used may affect the structure and function of DNA, interfere with the synthesis and repair of nucleic acids, or inhibit the synthesis and function of certain housekeeping proteins (such as microtubulin), and thus have a general cytotoxicity, with the disadvantages of high toxic side effects and easy drug resistance in clinical application.

PI3K small-molecule inhibitors have great potential and promise as a new class of molecular targeted drugs. Therefore, more PI3K inhibitors with novel structures, high biological activity and good drug-forming properties are needed for targeted therapy of tumors, as well as for anti-inflammatory or treatment of autoimmune diseases.

A new generation of PI3K small-molecule inhibitors showed significant inhibition against proliferation activity in a variety of tumor cells. However, there is a phenomenon of drug polymorphism, as one of the important factors affecting the quality and clinical efficacy of drugs. Different crystal forms can lead to differences in stability, absorption and bioavailability, thus affecting their clinical efficacy. Therefore, the polymorphic crystals of the fused ring compound and its preparation technology are of great importance for applications of the drug.

### Summary of the Disclosure

An object of the present disclosure is to provide a crystal form of a fused ring compound, a composition thereof, a preparation method thereof and uses thereof. The crystal form of the fused ring compound can be used for targeted therapy of tumors, as well as for anti-inflammatory or treatment of autoimmune diseases.

The present disclosure provides polymorphic forms of a fused ring compound.

The fused ring compound has a structural formula as follows.

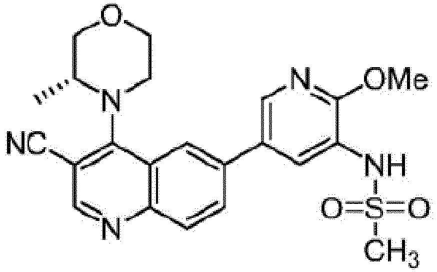

The polymorphic crystals comprise: crystal form I, having characteristic diffraction peaks at 2θ angles of about11.3°, 17.2°, 21.1° using X-ray diffraction; crystal form II, having characteristic diffraction peaks at 2θ angles of about 25.1°, 21.2°, 14.1° using X-ray diffraction; crystal form III, having characteristic diffraction peaks at 2θ angles of about 6.6°, 13.4° and 8.0° using X-ray diffraction; crystal form IV, having characteristic diffraction peaks at 2θ angles of about 11.8°, 13.3° and 16.7° using X-ray diffraction; or crystal form V, having characteristic diffraction peaks at 2θ angles of about 6.5°, 13.3° and 20.0° using X-ray diffraction, wherein the error in the 2θ angles is ±0.5°.

Further, crystal form I further has characteristic diffraction peaks at 2θ angles of about 11.3°, 17.2°, 21.1°, 22.7°, 18.5°, 13.8°using X-ray diffraction; crystal form II further has characteristic diffraction peaks at 2θ angles of about 25.1°, 21.2°, 14.1°, 16.0°, 7.0°, 18.4°using X-ray diffraction; crystal form III further has characteristic diffraction peaks at 2θ angles of about 6.6°, 13.4°, 8.0°, 20.0°, 21.1°, 10.5° using X-ray diffraction; crystal form IV further has characteristic diffraction peaks at 2θ angles of about 11.8°, 13.3°, 16.7°, 17.8°, 21.8°, 24.4° using X-ray diffraction; crystal form V further has characteristic diffraction peaks at 2θ angles of about 6.5°, 13.3°, 20.0°, 10.4°, 24.0°, 24.8° using X-ray diffraction, wherein the error in the 2θ angles is ±0.5°.

Specifically, crystal form I further has characteristic diffraction peaks at 2θ angles of 11.3°, 13.8°, 17.2°, 18.5°, 20.2°, 21.1°, 22.7°, 25.2°, 28.0°, 29.6°, 31.2°, 33.2° using X-ray diffraction; crystal form II further has characteristic diffraction peaks at 2θ angles of 5.7°, 7.0°, 9.9°, 11.5°, 12.2°, 14.1°, 16.0°, 17.3°, 18.4°, 21.2°, 22.9°, 25.1°, 31.7° using X-ray diffraction; crystal form III further has characteristic diffraction peaks at 2θ angles of 6.6°, 8.0°, 8.8°, 10.5°, 11.9°, 13.4°, 14.3°, 17.9°, 20.0°, 21.1°, 22.8° using X-ray diffraction; crystal form IV further has characteristic diffraction peaks at 2θ angles of 6.6°, 8.2°, 9.7°, 11.8°, 13.3°, 15.0°, 16.7°, 17.8°, 20.2°, 21.8°, 24.4°, 27.9°, 29.1°, 30.4° using X-ray diffraction; crystal form V further has characteristic diffraction peaks at 2θ angles of 6.5°, 8.0°, 10.4°, 13.3°, 16.2°, 17.7°, 20.0°, 21.0°, 24.0°, 24.8°, 26.7°, 29.1° using X-ray diffraction.

Another object of the present disclosure is to provide a composition comprising polymorphic forms of a fused ring compound for targeted therapy of tumors, and for anti-inflammatory or treatment of autoimmune diseases.

A composition comprising polymorphic forms of a fused ring compound, wherein crystal form I and crystal form IV account for 50% by weight or more of the crystal composition.

A composition comprising polymorphic forms of a fused ring compound, comprising one of crystal form I, crystal form II, crystal form III, crystal form IV and crystal form Vor any combination thereof, and wherein crystal form I and crystal form IV account for 50% by weight or more of the crystal composition.

Another object of the present disclosure is to provide a composition comprising polymorphic forms of a fused ring compound for targeted therapy of tumors, and for anti-inflammatory or treatment of autoimmune diseases.

A composition comprising polymorphic forms of a fused ring compound, wherein crystal form I and crystal form IV account for 80% by weight or more of the crystal composition.

A composition comprising polymorphic forms of a fused ring compound, comprising one of crystal form I, crystal form II, crystal form III, crystal form IV and crystal form V or any combination thereof, and wherein crystal form I and crystal form IV account for 80% by weight or more of the crystal composition.

Another object of the present disclosure is to provide a composition comprising polymorphic forms of a fused ring compound for targeted therapy of tumors, and for anti-inflammatory or treatment of autoimmune diseases.

A composition comprising polymorphic forms of a fused ring compound, wherein crystal form I and crystal form IV account for 90% by weight or more of the crystal composition. A composition comprising polymorphic forms of a fused ring compound, comprising one of crystal form I, crystal form II, crystal form III, crystal form IV and crystal form V or any combination thereof, and crystal form I and crystal form IV account for 90% by weight or more of the crystal composition.

Another object of the present disclosure is to provide a pharmaceutical composition comprising the above-mentioned crystal forms for targeted therapy of tumors, and for anti-inflammatory or treatment of autoimmune diseases.

A pharmaceutical composition comprising crystal form I, crystal form II, crystal form III, crystal form IV or crystal form V and a pharmaceutically acceptable excipient as components.

Another object of the present disclosure is to provide a method for preparing crystal form I, comprising adding a first organic solvent to the fused ring compound, heating to dissolve the fused ring compound, and adding a second organic solvent thereto, followed by stirring for crystallization, filtering and drying.

Further, the first organic solvent is one or more of dichloromethane, trichloromethane and tetrahydrofuran, and the amount of the first organic solvent is 3-5 times the weight of the fused ring compound. The second organic solvent is one or more of acetone, butanone, ethanol and ethyl acetate, and the amount of the second organic solvent is 6-12 times the weight of the fused ring compound.

Further, the stirring for crystallization is carried out at a temperature of 0-40°C for a time period of 4-15 hours, and the drying is carried out at a temperature of 60-150°C for a time period of 4-15 hours.

Another object of the present disclosure is to provide a method for preparing crystal form IV, comprising dissolving the fused ring compound in an organic solvent and/or water under heating, followed by stirring for crystallization, filtering and drying.

Further, the organic solvent and/or water is a mixture of acetonitrile and water, and the amount of acetonitrile is 30-90 times the weight of the fused ring compound and the amount of water is 0-20 times the weight of the fused ring compound.

Further, the stirring for crystallization is carried out at a temperature of 0-40°C for a time period of 0.5-30 hours, and the drying is carried out at a temperature of 60-150°C for a time period of 1-20 hours.

Another object of the present disclosure is to provide use of the above crystal forms in manufacture of a medicament for inhibiting phosphatidylinositol 3-kinase.The medicament can be used for targeted therapy of tumors, as well as for anti-inflammatory or treatment of autoimmune diseases.

Use the above crystal forms in manufacture of a medicament for inhibiting phosphatidylinositol 3-kinase, and the medicament is an anti-tumor drug.

Further, the tumor is selected from the group consisting of brain cancer, head and neck cancer, esophageal cancer, lung cancer, liver cancer, stomach cancer, kidney cancer, pancreatic cancer, prostate cancer, colorectal cancer, ovarian cancer, breast cancer, thyroid cancer, skin cancer, leukemia, myelodysplastic syndrome, sarcoma, osteosarcoma and rhabdomyosarcoma.

Further, the medicament is an anti-inflammatory drug or a drug for treatment of autoimmune diseases.

Further, the anti-inflammatory drug is a drug for treatment of chronic obstructive pulmonary disease or asthma, and the autoimmune disease is rheumatoid arthritis, psoriasis or systemic lupus erythematosus.

The beneficial effects of the present disclosure include:
Crystal forms I, II, III, IV and V of the present application all have obvious diffraction characteristic peaks for the respectivecrystal form, which can overcome the technical defect of amorphous form having no regular crystal structure. The stability of crystal form I, crystal form II and crystal form IV of the present disclosure under high humidity test and high temperature test for 5 days is significantly better than that of the amorphous form in the prior art (CN 201610235304.5). Crystal form I and crystal form IV of the present disclosure have good solubility and stability in different solutions, are easily absorbed by human body, and possess excellent effects in treating diseases.

### Brief Description of the Drawings

FIG. 1A shows an X-ray diffraction pattern of crystal form I in the present disclosure.
FIG. 1B shows an X-ray diffraction peak data plot of crystal form I in the present disclosure.
FIG. 1C shows a DSC plot of crystal form I in the present disclosure.
FIG. 2A shows an X-ray diffraction pattern of crystal form II obtained in Example 7.
FIG. 2B shows an X-ray diffraction peak data plot of crystal form II obtained in Example 7.
FIG. 3A shows an X-ray diffraction pattern of crystal form III obtained in Example 9.
FIG. 3B shows an X-ray diffraction peak data plot of crystal form III obtained in Example 9.
FIG. 4A shows an X-ray diffraction pattern of crystal form V obtained in Example 10.
FIG. 4B shows an X-ray diffraction peak data plot of crystal form V obtained in Example 10.
FIG. 5A shows an X-ray diffraction pattern of crystal form IV in the present disclosure.
FIG. 5B shows an X-ray diffraction peak data plot of crystal form IV in the present disclosure.
FIG. 5C shows a DSC plot of crystal form IV in the present disclosure.
FIG. 6 shows an X-ray diffraction pattern of crystal form I obtained in Example 6.
FIG. 7 shows an X-ray diffraction pattern of crystal form II obtained in Example 8.
FIG. 8 shows an X-ray diffraction pattern of the amorphous form obtained in Example 11.
FIG. 9 shows an X-ray diffraction pattern of the amorphous form after a high humidity test for 5 days in Example 12.
FIG. 10 shows an X-ray diffraction pattern of crystal form IV after a high humidity test for 5 days in Example 12.
FIG. 11 shows an X-ray diffraction pattern of crystal form I after a high humidity test for 5 days in Example 12.
FIG. 12 shows an X-ray diffraction pattern of crystal form II after a high humidity test for 5 days in Example 12.
FIG. 13 shows an X-ray diffraction pattern of the amorphous form after a high temperature test for 5 days in Example 13.
FIG. 14 shows an X-ray diffraction pattern of crystal form IV after a high temperature test for 5 days in Example 13.
FIG. 15 shows an X-ray diffraction pattern of crystal form I after a high temperature test for 5 days in Example 13.
FIG. 16 shows an X-ray diffraction pattern of crystal form II after a high temperature test for 5 days in Example 13.
FIG. 17 shows an X-ray diffraction pattern of crystal form IV after an accelerated test for 30 days in Example 14.
FIG. 18 shows an X-ray diffraction pattern of crystal form I after an accelerated test for 30 days in Example 14.
Figure 19 shows an X-ray diffraction pattern of crystal form II after an accelerated test for 30 days in Example 14.

### Detailed Description of the Disclosure

The present disclosure is further described below in connection with preferred embodiments for better understandings for the disclosure. It should be understood by those skilled in the art that the detailed description below is only illustrative and not limiting, while it should not be used to limit the protection scope of the present disclosure.

All numerical designations of the present disclosure (e.g., temperature, time, concentration, and weight, including ranges for each of these) may generally be approximated by varying (+) or (-) in appropriate increments of 0.1 or 1.0. All numerical designations are understood to be preceded by the term "about".

A crystal form of a fused ring compound and a preparation method thereof and uses thereof, wherein the fused ring compound has a structure of the following formula:

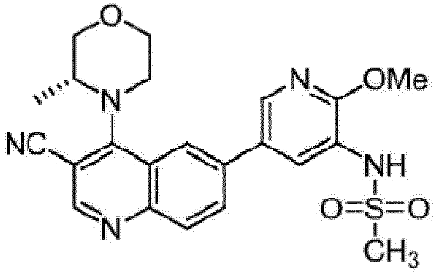

Crystal form I, having characteristic diffraction peaks at 2θ angles of about 11.3°, 17.2°, 21.1° using X-ray diffraction; crystal form II, having characteristic diffraction peaks at 2θ angles of about 25.1°, 21.2°, 14.1° using X-ray diffraction; crystal form III, having characteristic diffraction peaks at 2θ angles of about 6.6°, 13.4° and 8.0° using X-ray diffraction; crystal form IV, having characteristic diffraction peaks at 2θ angles of about 11.8°, 13.3° and 16.7° using X-ray diffraction; or crystal form V, having characteristic diffraction peaks at 2θ angles of about 6.5°, 13.3° and 20.0° using X-ray diffraction; wherein the error in the 2θ angles is ±0.5°.

Further, crystal form I further has characteristic diffraction peaks at 2θ angles of about 11.3°, 17.2°, 21.1°, 22.7°, 18.5°, 13.8° using X-ray diffraction; crystal form II further has characteristic diffraction peaks at 2θ angles of about 25.1°, 21.2°, 14.1°, 16.0°, 7.0°, 18.4° using X-ray diffraction; crystal form III further has characteristic diffraction peaks at 2θ angles of about 6.6°, 13.4°, 8.0°, 20.0°, 21.1°, 10.5° using X-ray diffraction; crystal form IV further has characteristic diffraction peaks at 2θ angles of about 11.8°, 13.3°, 16.7°, 17.8°, 21.8°, 24.4° using X-ray diffraction; crystal form V further has characteristic diffraction peaks at 2θ angles of about 6.5°, 13.3°, 20.0°, 10.4°, 24.0°, 24.8° using X-ray diffraction; wherein the error in the 2θ angles is ±0.5°.

### Example 1

Preparation of crystal form I:
10g of the fused ring compound prepared in Example 11 and 30g of dichloromethane were added to a reaction flask, heated to dissolve, and further added with 90g of ethanol, concentrated to a remaining weight of about 100g, followed by stirring for crystallization at 20°C for 4 hours, then filtered, and dried at 100°C to obtain crystal form I.

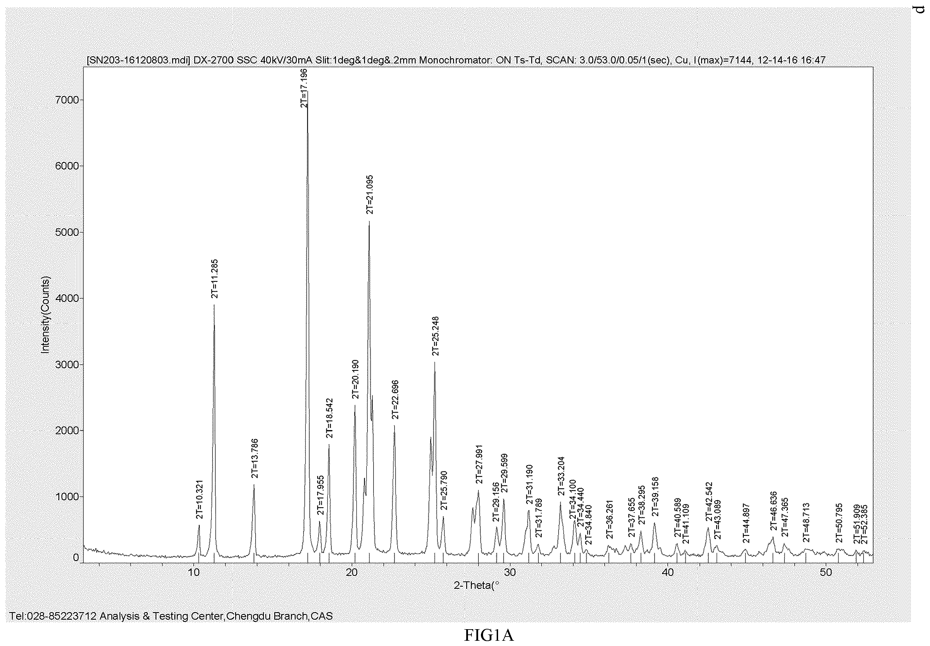

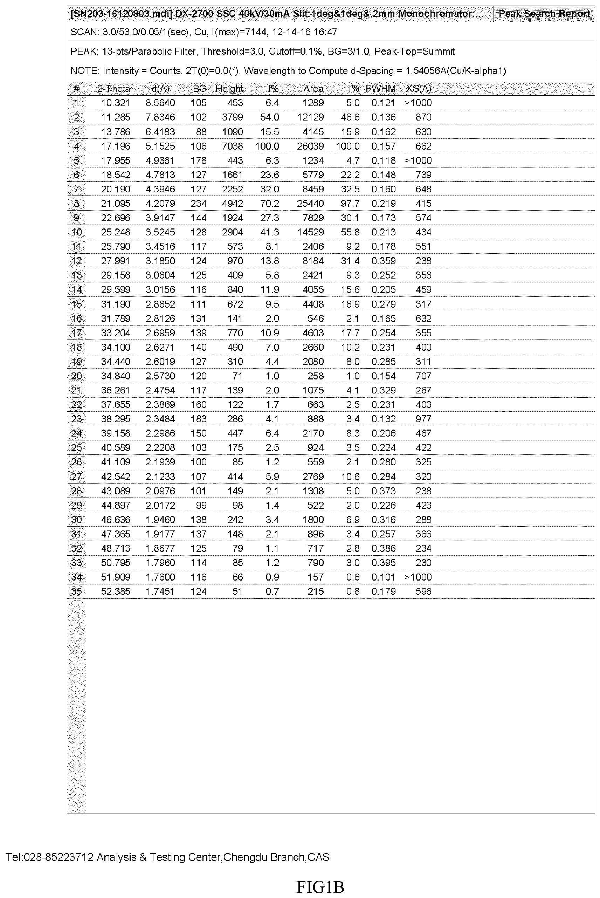

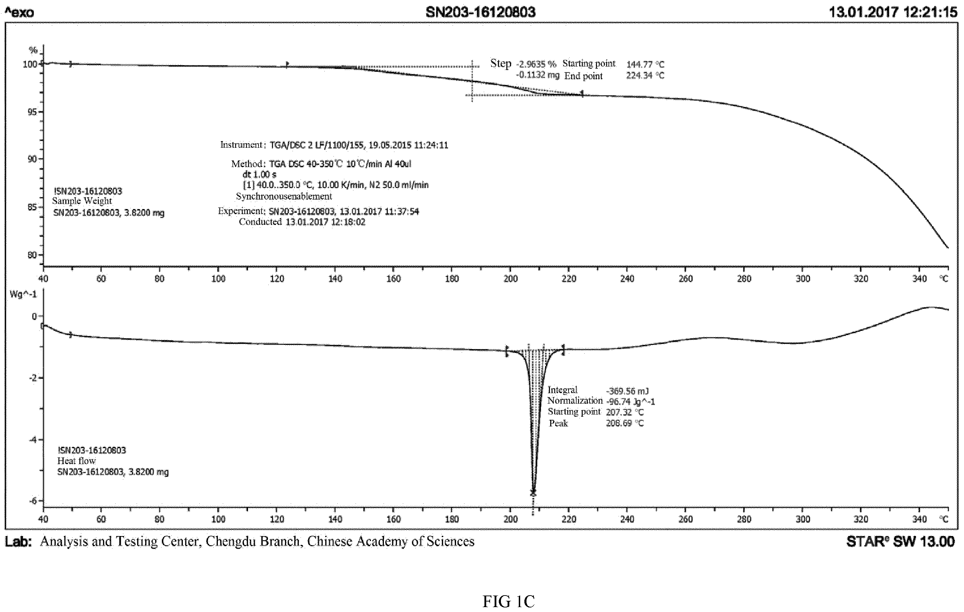

The crystal form I prepared by the above process had a melting point of 207-209°C.The X-ray diffraction pattern showed characteristic diffraction peaks at 2θ angles of 11.3°, 13.8°, 17.2°, 18.5°, 20.2°, 21.1°, 22.7°, 25.2°, 28.0°, 29.6°, 31.2°, and 33.2° (see Figs. 1A and 1B). Fig. 1C showed thermal analysis plot of crystal form I. TGA showed that crystal form I started to lose weight at about 145°C, while DSC plot shows an absorption peak at about 209°C.

### Example 2

The solubilities (µg/mL) of crystal form I in different environments are shown in Table 1.

**Table 1**

| Solvent | Ultrasonic | R.T. (25°C) | 37°C | 75°C | Stability |
|---|---|---|---|---|---|
| Water | 4.63 | 3.84 | 3.87 | 2.24 | Stable within 24 hours |
| pH 1.2 hydrochloric acid solution | 294.60 | 275.20 | 404.30 | 867.60 | Stable within 24 hours |
| pH 4.5 acetic acid-sodium acetate solution | 1.56 | 1.29 | 1.67 | 1.55 | Stable within 24 hours |
| pH 6.8 PBS solution | 2.79 | 3.58 | 1.51 | 1.41 | Stable within 24 hours |
| Simulated gastric fluid (SGF) | 119.65 | 99.81 | 167.38 | 431.94 | Stable within 24 hours |
| Simulated fasting intestinal fluid | 4.76 | 2.07 | 2.87 | 8.64 | Stable within 24 hours |
| (FaSSIF) | | | | | |
| Simulated postprandial intestinal fluid (FeSSIF) | 9.46 | 8.09 | 11.35 | 18.92 | Stable within 24 hours |

### Example 3

Preparation of crystal form IV:
10g of the fused ring compound prepared in Example 11 and 300g of acetonitrile were added to a reaction flask, heated to dissolve, followed by stirring for crystallization at 25°C for 4 hours, then filtered, and dried at 80°C for 10 hours to obtain crystal form IV.

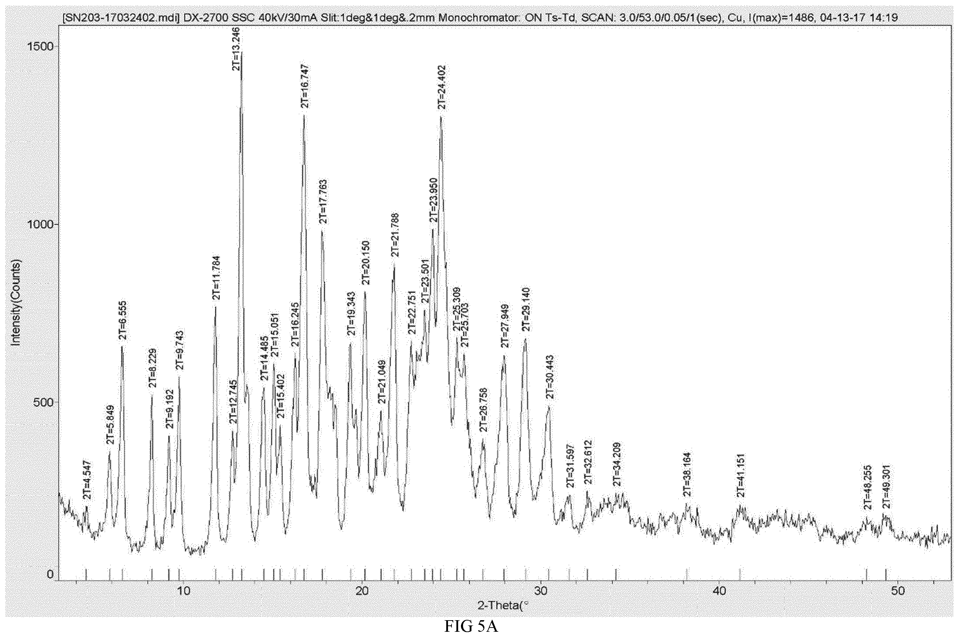

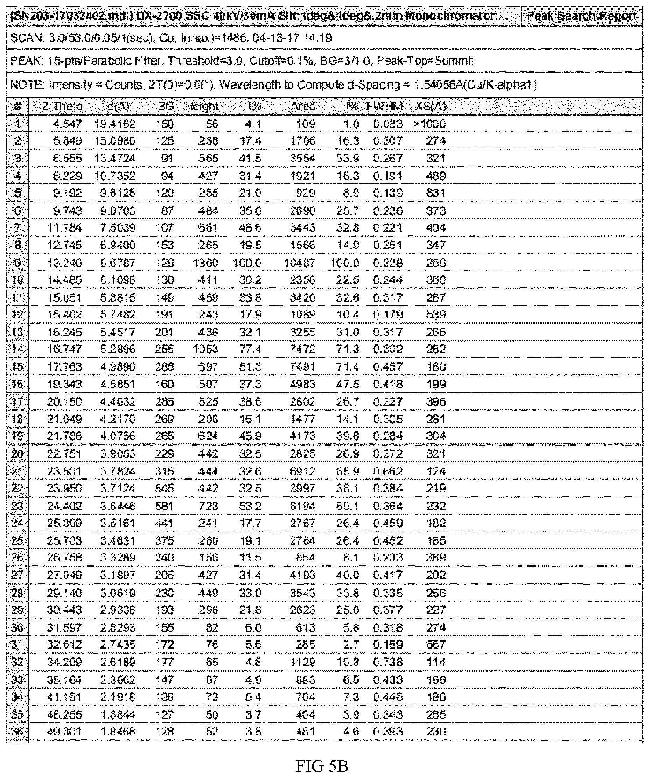

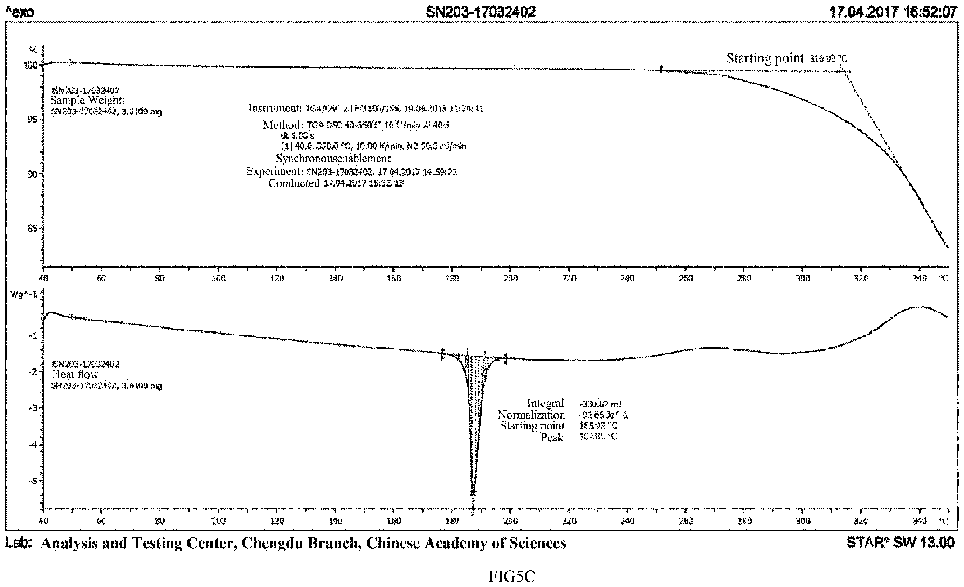

The crystal form IV prepared by the above process had a melting point of 185-188°C. The X-ray diffraction pattern showed characteristic diffraction peaks at 2θ angles of 6.6°, 8.2°, 9.7°, 11.8°, 13.3°, 15.0°, 16.7°, 17.8°, 20.2°, 21.8°, 24.4°, 27.9°, 29.1°, 30.4° (see Figs. 5A and 5B). Fig.5C showed a thermal analysis plot of crystal form IV TGA showed that crystal form IV started to lose weight at about 250°C, while DSC plot shows an absorption peak at about 188°C.

### Example 4

Preparation of crystal form IV:
10g of the fused ring compound prepared in Example 11 and 200g of acetonitrile were added to a reaction flask, heated to dissolve, then added with 50g of water, followed by stirring for crystallization at 5°C for 20 hours, and then filtered, and dried at 120°C for 2 hours to obtain crystal form IV.

The crystal form IV prepared by the above process had a melting point of 185-187°C. The X-ray diffraction pattern showed characteristic diffraction peaks at 2θ angles of 6.6°, 8.2°, 9.7°, 11.8°, 13.2°, 15.0°, 16.7°, 17.8°, 20.2°, 21.8°, 24.4°, 27.9°, 29.1°, and 30.4°.

### Example 5

The solubilities (µg/mL) of crystal form IV in different environments are shown in Table 2.

**Table 2**

| Solvent | Ultrasonic | R.T. (25°C) | 37°C | 75°C | Stability |
|---|---|---|---|---|---|
| Water | 2.96 | 2.68 | 2.39 | 2.98 | Stable within 24 hours |
| pH 1.2 | 1289.40 | 1283.50 | 1431.40 | 1516.50 | Stable within 24 |
| hydrochloric acid solution | | | | | hours |
| pH 4.5 acetic acid-sodium acetate solution | 3.04 | 2.75 | 3.04 | 3.45 | Stable within 24 hours |
| pH 6.8 PBS solution | 2.48 | 2.12 | 2.18 | 2.62 | Stable within 24 hours |
| Simulated gastric fluid (SGF) | 570.49 | 533.36 | 655.37 | 766.58 | Stable within 24 hours |
| Simulated fasting intestinal fluid (FaSSIF) | 5.43 | 6.05 | 4.70 | 5.12 | Stable within 24 hours |
| Simulated postprandial intestinal fluid (FeSSIF) | 29.70 | 31.70 | 26.02 | 28.48 | Stable within 24 hours |

### Example 6

Preparation of crystal form I:
1.0g of the fused ring compound prepared in Example 11 was added to a flask, which is further added with 30mL of ethanol as a solvent, heated at an oil bath temperature of 85°C, stirred and dissolved to clear, then left to stand at room temperature, cooled for crystallization, and then filtered and collected for solids, and dried under vacuum at room temperature.

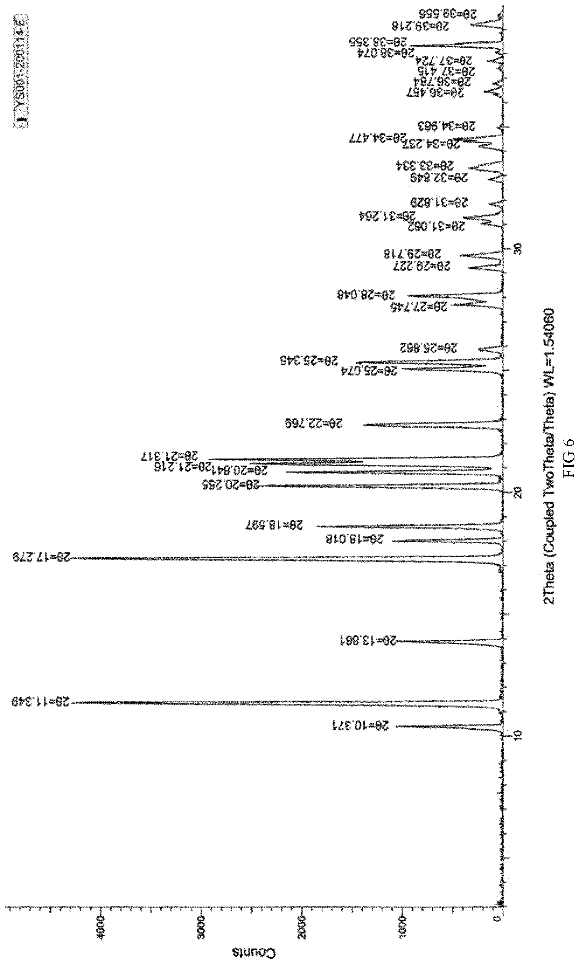

The X-ray diffraction pattern of crystal form I prepared by the above process showed characteristic diffraction peaks at 2θ angles of 10.4°, 11.3°, 13.9°, 17.3°, 18.0°, 18.6°, 20.3°, 20.8°, 21.3°, 22.8°, 25.3°, 28.0°, 29.7°, and 31.3° (see Fig. 6).

### Example 7

Preparation of crystal form II:
4.0g of the fused ring compound prepared in Example 11 was added with 12.0g of dichloromethane, heated to dissolve, then added with 36.0g of anhydrous methanol, stirred for 16 hours at 15°C and filtered. The filter cake was washed with a small amount of methanol, dried at 60°C under reduced pressure, and baked to obtain 3.90g of a light yellow solid.

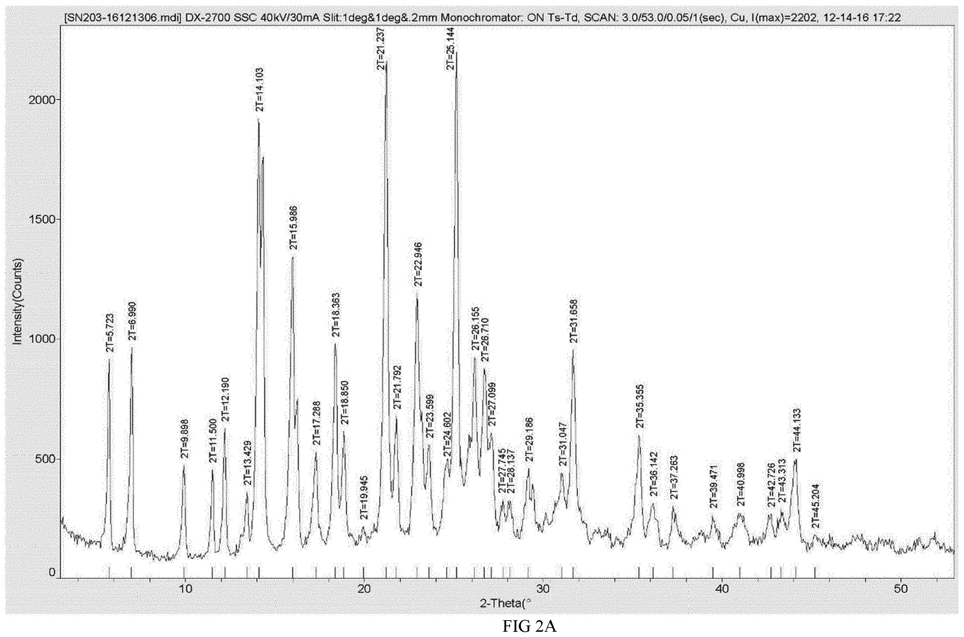

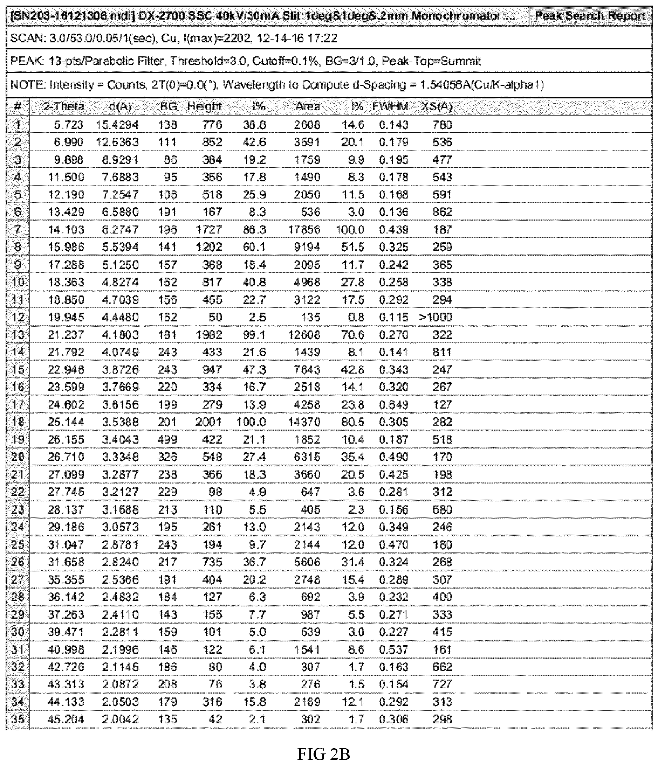

The characterization data of crystal form II prepared by the above process were shown in Figs. 2A and 2B. The X-ray diffraction patterns showed characteristic diffraction peaks at 2θ angles of 5.7°, 7.0°, 9.9°, 11.5°, 12.2°, 14.1°, 16.0°, 17.3°, 18.4°, 21.2°, 22.9°, 25.1°, and 31.7° (see Fig. 2A).

### Example 8

Preparation of crystal form II:
1.0g of the fused ring compound prepared in Example 11 was added to a flask, which is further added with 45mL of solvent (EA:MeOH=1:1), heated at oil bath temperature of 70°C, stirred and dissolved to clear, then left to stand at room temperature, cooled for crystallization, then filtered and collected for solids, and dried under vacuum at room temperature.

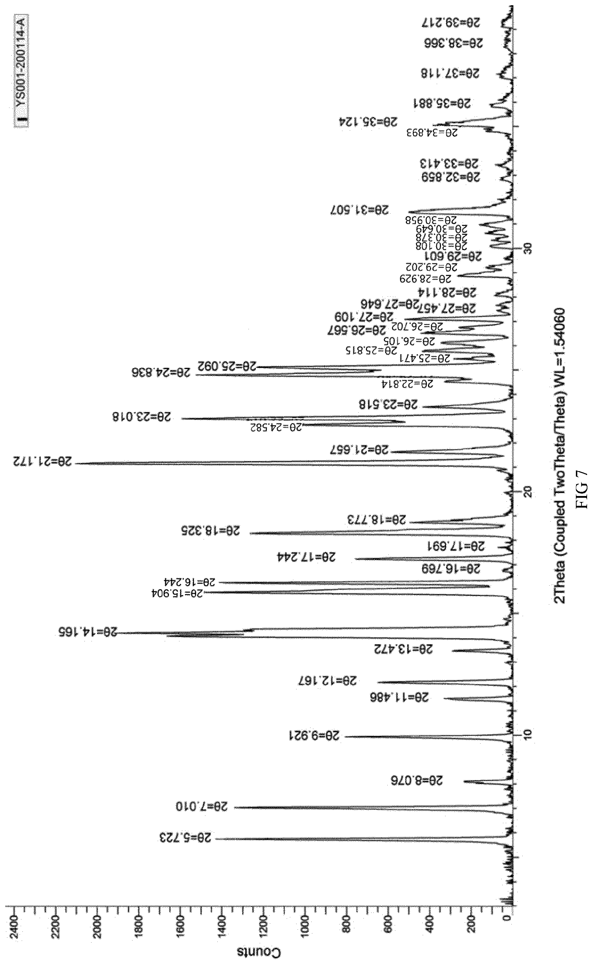

The X-ray diffraction patterns of crystal form II prepared by the above process showed characteristic diffraction peaks at 2θ angles of 5.7°, 7.0°, 9.9°, 11.5°, 12.2°, 14.2°, 15.9°, 17.2°, 18.3°, 21.2°, 23.0°, 25.1°, and 31.5° (see Fig.7).

### Example 9

Preparation of crystal form III:
4.0g of the fused ring compound prepared in Example 11 was added with 200mL of ethyl acetate, heated to reflux until the solid dissolved, cooled down for crystallization, stirred at 15°C for 16 hours and filtered. The filter cake was dried at 60°C under reduced pressure and dried to obtain 3.55g of a light yellow solid.

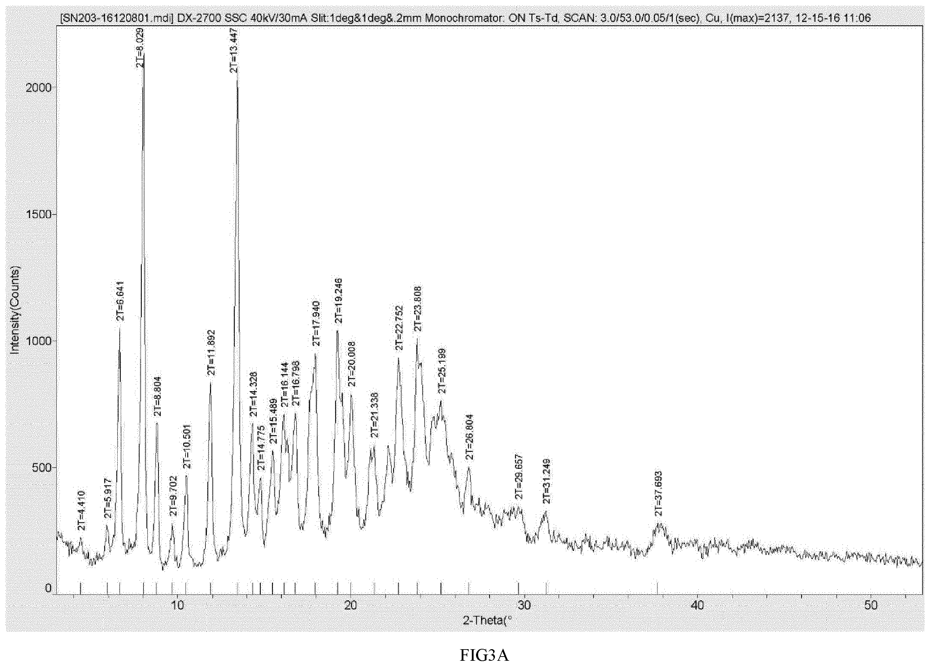

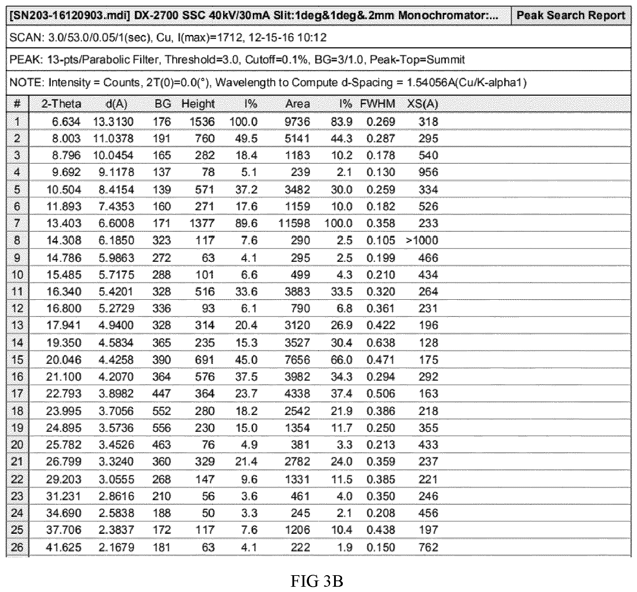

The characterization data of crystal form III prepared by the above process were shown in Figs. 3A and 3B. The X-ray diffraction patterns showed characteristic diffraction peaks at 2θ angles of 6.6°, 8.0°, 8.8°, 10.5°, 11.9°, 13.4°, 14.3°, 17.9°, 20.0°, 21.1°, and 22.8° (see Fig. 3A).

### Example 10

Preparation of crystal form V:
1.0 g of the fused ring compound prepared in Example 11 was added with 6.0 g of tetrahydrofuran, heated to reflux until the solid dissolved, cool down for crystallization, stirred at 15°C for 16 hours and filtered. The filter cake was dried at 60°C under reduced pressure and dried to obtain 0.29 g of a light yellow solid.

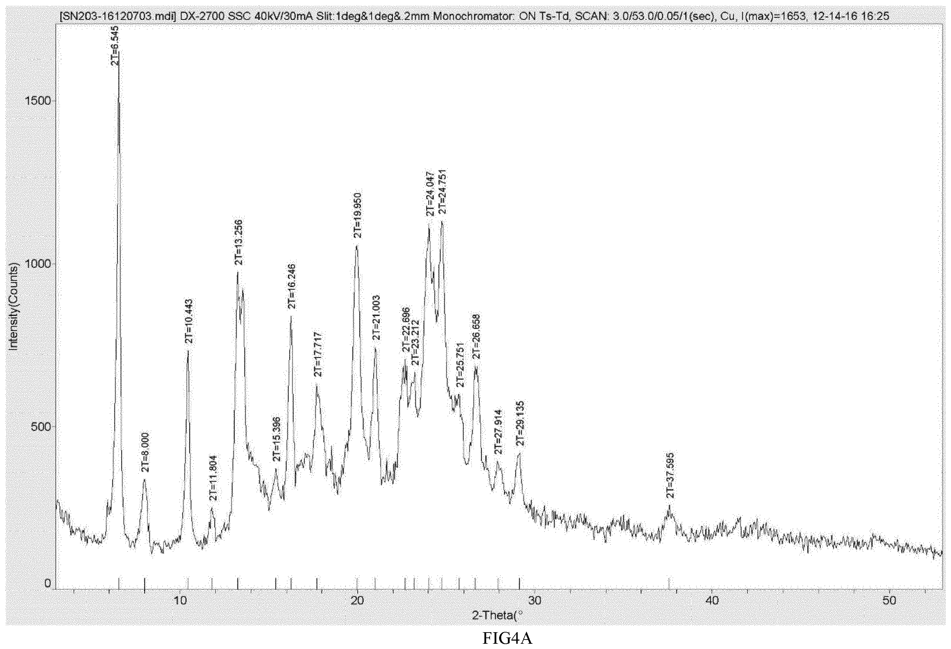

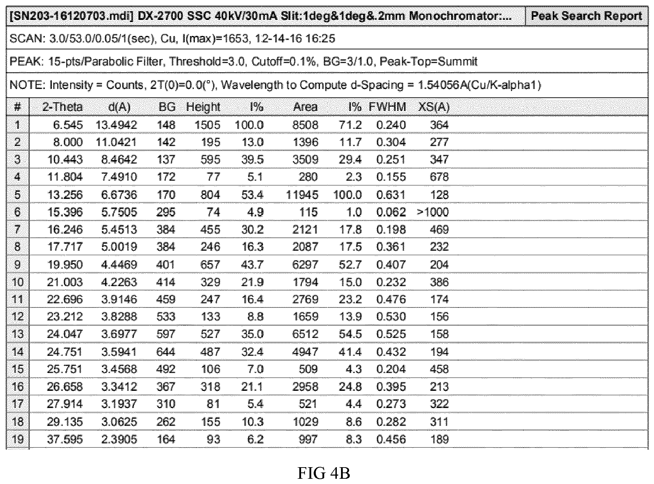

The characterization data of crystal form V prepared by the above process were shown in Figs. 4A and 4B. The X-ray diffraction patterns showed characteristic diffraction peaks at 2θ angles of 6.5°, 8.0°, 10.4°, 13.3°, 16.2°, 17.7°, 20.0°, 21.0°, 24.0°, 24.8°, 26.7°, and 29.1° (see Fig. 4A).

### Example 11

Reference was made to the literature (CN 201610235304.5) for the preparation of the fused ring compound: (R)-N-(5-(3-cyano-4-(3-methylmorpholinyl)quinolin-6-yl)-2-methoxypyridin-3-yl)methanesulfonamide.

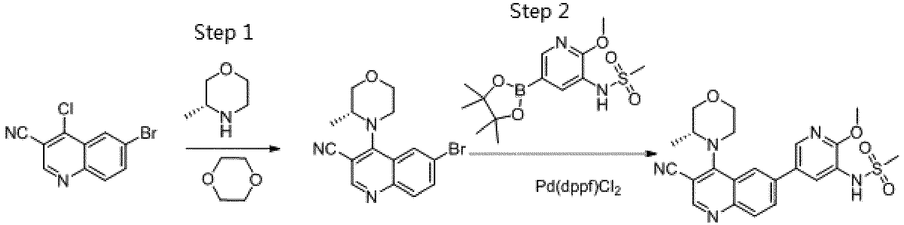

### Step 1:

6-bromo-4-chloro-quinoline-3-carbonitrile (1.605 g, 6.0 mmol) and (R)-3-methylmorpholine (1.821 g, 18.0 mmol, 3eq) were mixed in dioxane (30.0 mL) and stirred at 100 °C. Upon the reaction was completed, the resultant was concentrated under vacuum, and the concentrate was diluted with water (60 mL) and extracted with ethyl acetate (60 mL×3).The organic layers were combined, washed sequentially with water (20 mL) and saline solution (20 mL), dried over anhydrous sodium sulfate, filtered, concentrated and purified by fast column chromatography (silica gel, petroleum ether/ethyl acetate = 3:1, v/v) to give a pale yellow solid of (R)-6-bromo-4-(3-methylmorpholinyl)quinoline-3-carbonitrile (1.566 g, 78.5% yield).

¹HNMR (400 MHz, CDCl₃) δ 8.81 (s, 1H), 8.30 (d, *J=* 2.1 Hz, 1H), 7.96 (d, J = 8.9 Hz, 1H), 7.86 (dd, *J=* 8.9, 2.2 Hz, 1H), 4.13 - 4.05 (m, 2H), 4.05 - 3.97 (m, 1H), 3.91 - 3.83 (m, 1H), 3.77 (d, J = 9.8 Hz, 1H), 3.66 (dd, *J=* 11.9, 5.9 Hz, 1H), 3.33 - 3.26 (m, 1H), 1.14 (d, *J =* 6.4 Hz, 3H).

### Step 2 :

A mixture of (R)-6-bromo-4-(3-methylmorpholinyl)quinoline-3-carbonitrile (1.0 g, 3 mmol), N-(2-methoxy-5-(4,4,5,5-tetramethyl-1,3,2-dioxaborolan-2-yl)pyridin-3-yl)methanesulfonamide (1.18 g, 3.6 mmol, 1.2 eq) and 2 N aqueous potassium carbonate solution (4.5 mL, 3.0 eq) in dioxane (20 mL) was degassed and then [1,1'-bis(diphenylphosphino)ferrocene]palladium dichloride (110 mg, 0.15 mmol, 0.05 eq) was added. The resulting reaction mixture was degassed and back-charged with nitrogen (for three cycles) and then stirred for 5 hours at 100 °C under nitrogen atmosphere. The reaction mixture was cooled to room temperature, diluted with (30 mL) water, and extracted with ethyl acetate (30 mL × 3).The organic layers were combined and washed with saline solution (30 mL), dried over anhydrous sodium sulfate, filtered, concentrated and purified by fast column chromatography (silica gel, dichloromethane/methanol = 200:1, v/v) to give a white solid (1.056 g, 77.3% yield).

¹H NMR (400 MHz, DMSO) δ 9.47 (s, 1H), 8.86 (s, 1H), 8.45 (d, *J* = 2.0 Hz, 1H), 8.29 (s, 1H), 8.19 (dd, *J =* 8.8, 1.3 Hz, 1H), 8.10 (d, *J =* 8.7 Hz, 1H), 8.05 (d, *J* = 2.0 Hz, 1H), 4.14 - 4.04 (m, 2H), 4.00 (s, 3H), 3.99 - 3.93 (m, 1H), 3.90 - 3.82 (m, 1H), 3.80 - 3.70 (m, 1H), 3.62 (dd, *J* = 10.9, 3.6 Hz, 1H), 3.27 (d, *J* = 13.6 Hz, 1H), 3.14 (s, 3H), 1.10 (d, *J* = 6.2 Hz, 3H).

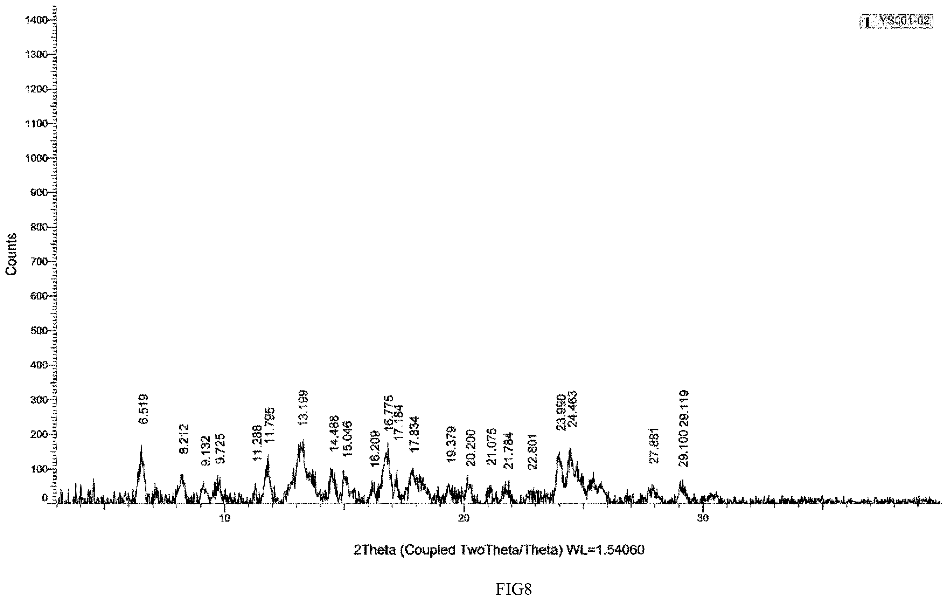

XRD of the resulting white solid is shown in Fig. 8, from which it can be seen that (R)-N-(5-(3-cyano-4-(3-methylmorpholinyl)quinolin-6-yl)-2-methoxypyridin-3-yl)methanesulfonamide obtained using the preparation method of literature(CN 201610235304.5) was amorphous.

### Example 12

### Evaluation on effect of crystal form on stability -high humidity test for 5 days

The amorphous form obtained in Example 11, crystal form IV obtained in Example 3, crystal form I obtained in Example 6, and crystal form II obtained in Example 8 were each taken 30 mg, in triplicate, put into a penicillin bottle, which is transferred, without being sealed, at room temperature into a desiccator with saturated potassium nitrate at the bottom, and the samples were taken after 5 days and tested by XRD

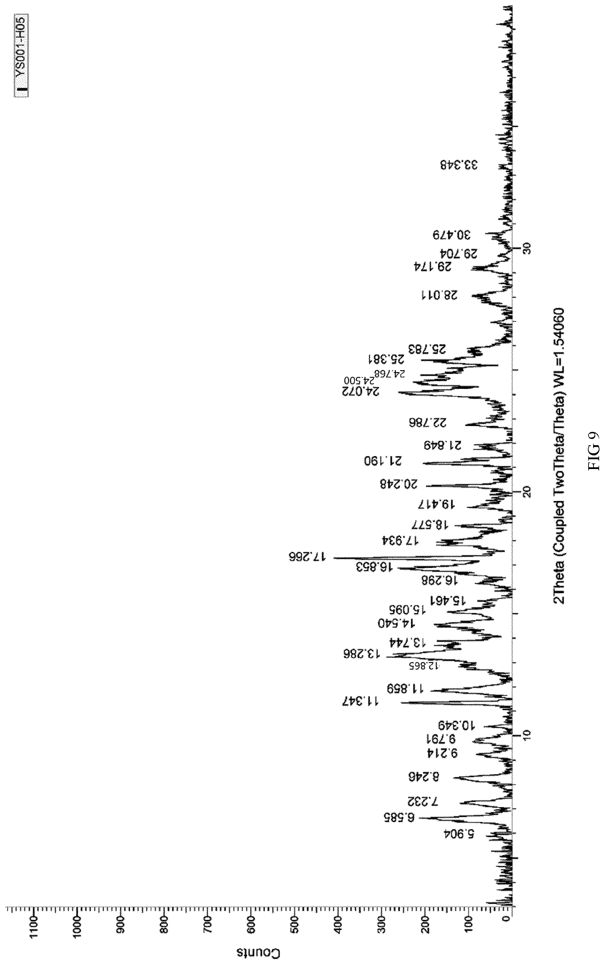

The XRD of the amorphous form after high humidity test for 5 days is shown in Fig. 9. Compared with that before the high humidity test (Fig. 8), there were obvious new peaks at 2θ angles of 7.2°, 10.3°, 11.3°, 16.9°, 17.3°, and 21.2°, with large changes in peak shape and peak intensity, indicating obvious crystal transformation in the amorphous form.

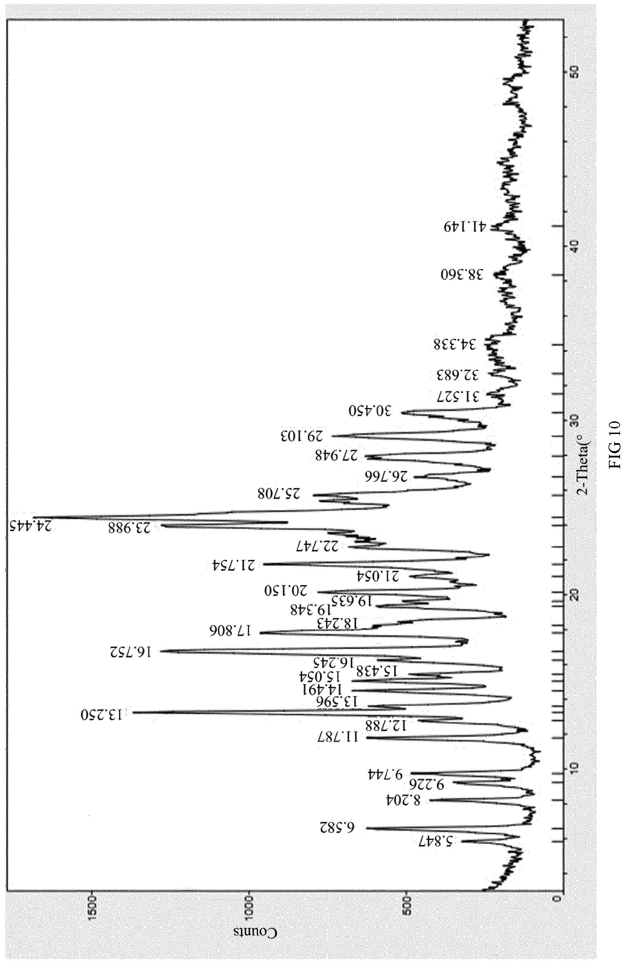

The XRD of crystal form IV after high humidity test for 5 days is shown in Fig. 10, which indicates barely no change for crystal form IV as compared to that before the high humidity test(Fig. 5A).

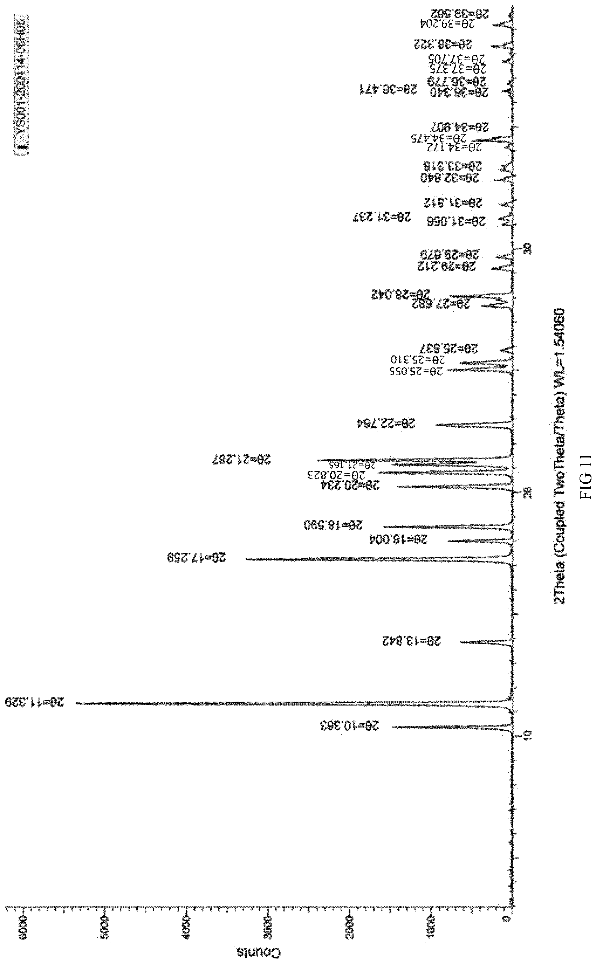

The XRD of crystal form I after high humidity test for 5 days is shown in Fig. 11, which indicates barely no change for crystal form I compared to that before the high humidity test(Fig. 6).

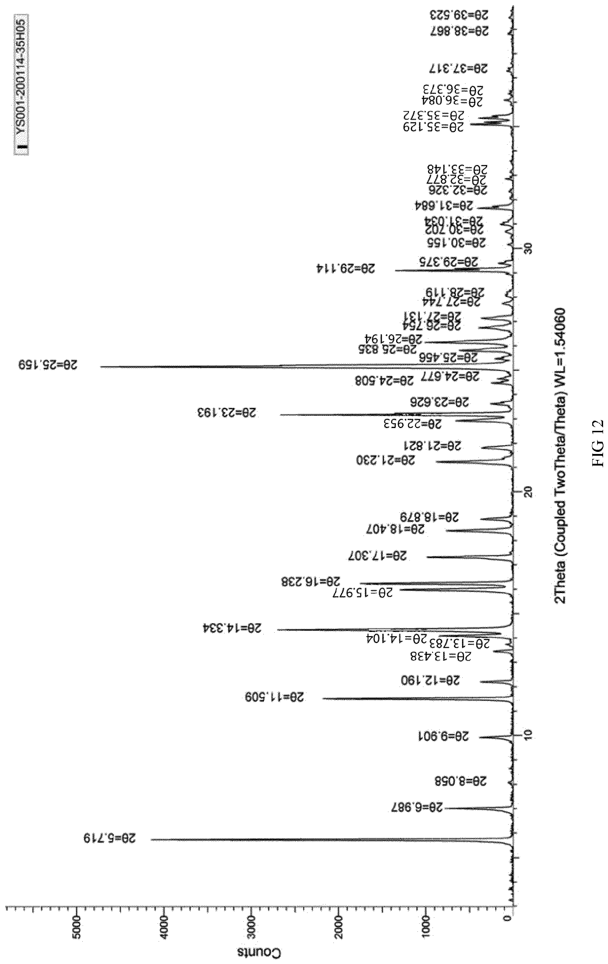

The XRD of crystal form II after high humidity test for 5 days is shown in Fig. 12, which indicates barely no change for crystal form II as compared to that before the high humidity test(Fig. 7).

### Example 13

### Evaluation on effect of crystal form on stability -high temperature test for 5 days

The amorphous form obtained in Example 11, crystal form IV obtained in Example 3, crystal form I obtained in Example 6, and crystal form II obtained in Example 8 were each taken 30 mg, in triplicate, put into a penicillin bottle, which is transferred, without being sealed, to QG 2003 ba incubation and drying oven, of which the temperature was adjusted to 60°C. The samples were taken after 5 days and tested by XRD

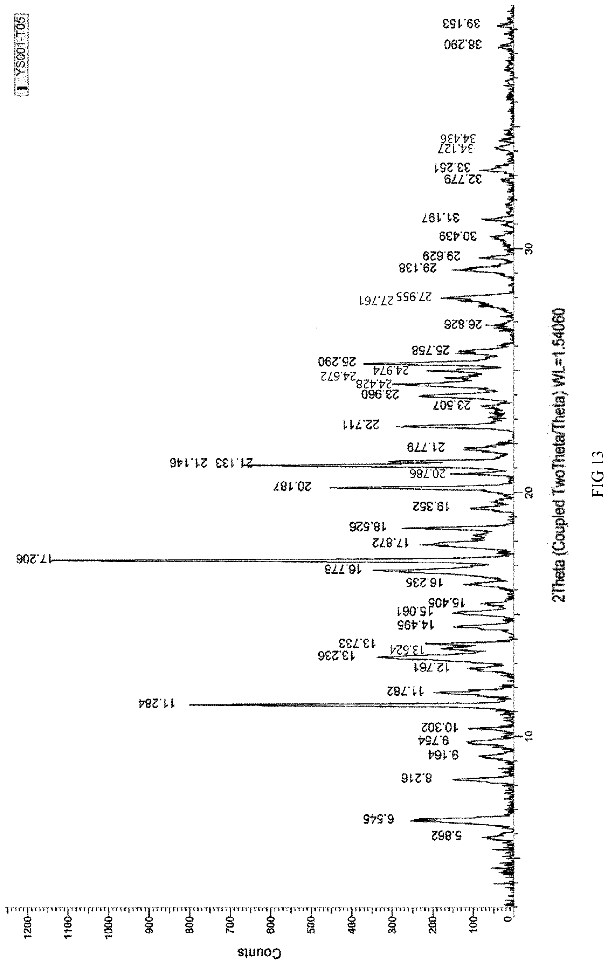

The XRD of the amorphous form after high temperature test for 5 days is shown in Fig. 13. Compared with that before the high temperature test (Fig. 8), there were obvious new peaks at 2θ angles of 11.3°, 17.2°, 18.5°, 20.2°, 21.1°, 22.7°, 25.3°, with large changes in peak shape and peak intensity, indicating obvious crystal transformation in the amorphous form.

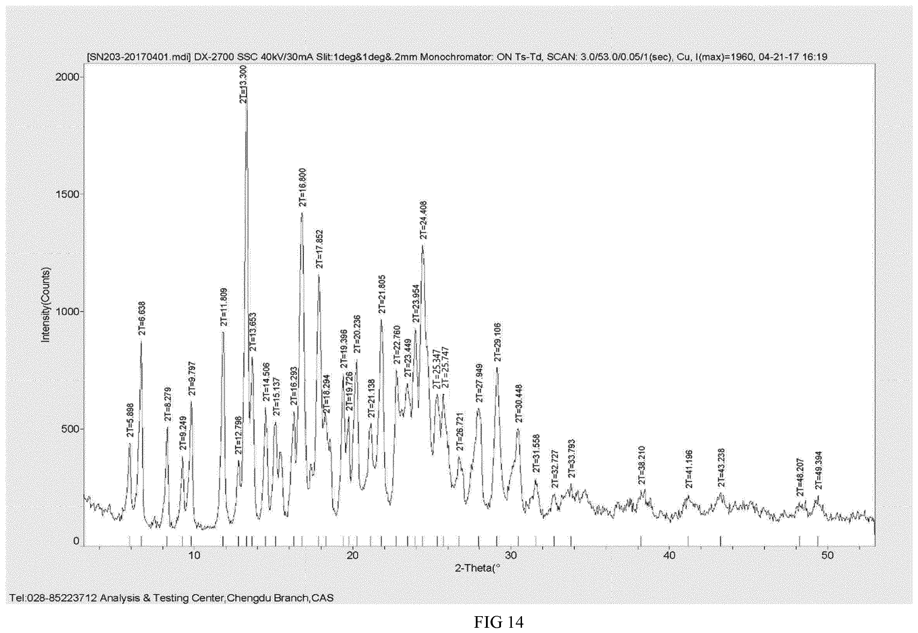

The XRD of crystal form IV after high temperature test for 5 days is shown in Fig. 14, which indicates barely no change for crystal form IV as compared to that before the high temperature test (Fig. 5A).

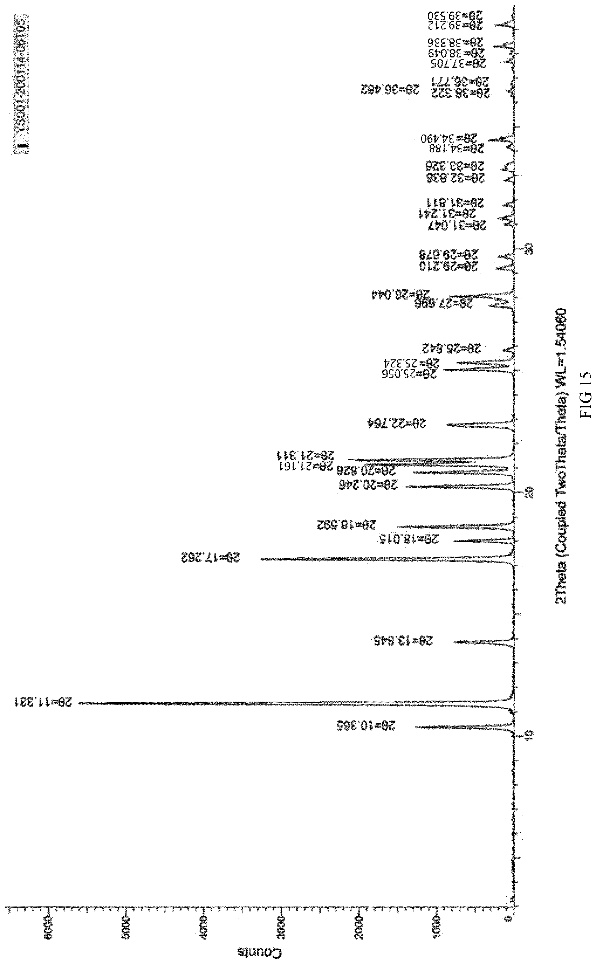

The XRD of crystal form I after high temperature test for 5 days is shown in Fig. 15, which indicates barely no change for crystal form I as compared to that before the high temperature test (Fig. 6).

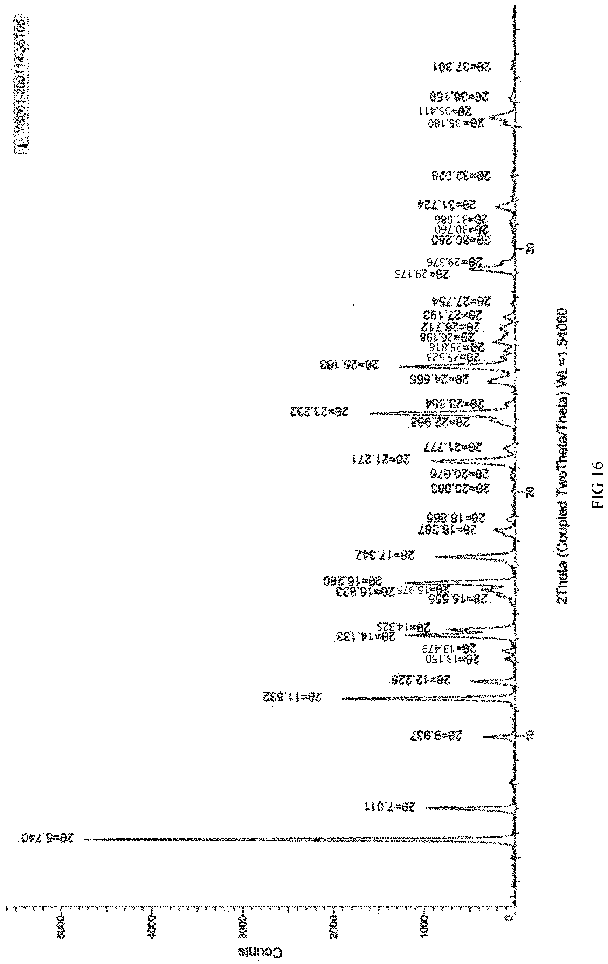

The XRD of crystal form II after high temperature test for 5 days is shown in Fig. 16, which indicates barely no change for crystal form II as compared to that before the high temperature test(Fig. 7).

### Example 14

### Evaluation for accelerated stability of crystal forms I, II and IV

Crystal form IV obtained in Example 3, crystal form I obtained in Example 6, and crystal form II obtained in Example 8 were each taken 30 mg, in triplicate, put into sealed bags, which is transferred into BPN-80CH CO₂ incubator, of which the temperature was set at 40°C. The samples were taken after 30 days and tested by XRD

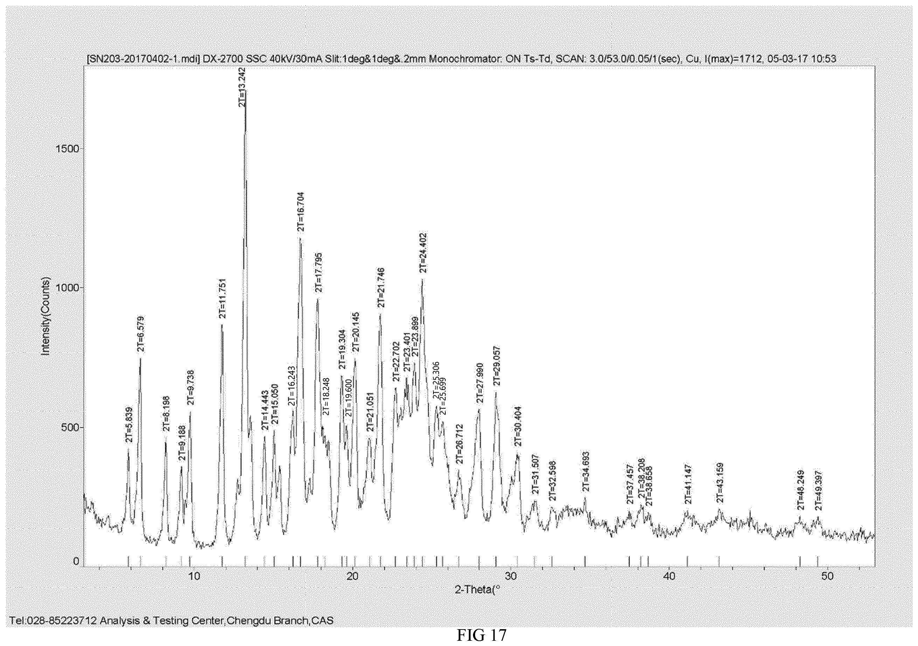

The XRD of crystal form IV after the accelerated stability test for 30 days is shown in Fig. 17, which indicates barely no change for crystal form IV as compared to that before the accelerated test(Fig. 5A).

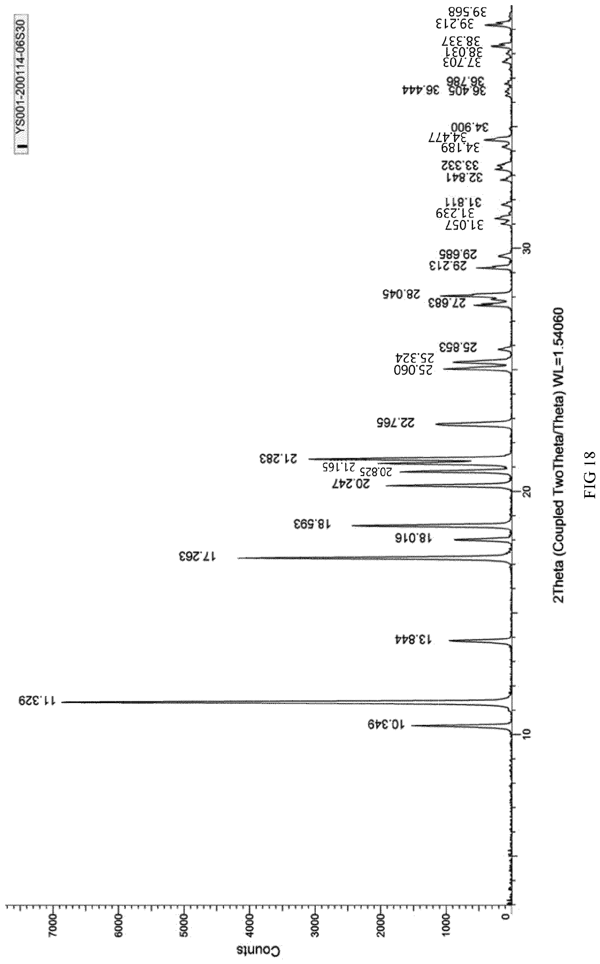

The XRD of crystal form I after the accelerated stability test for 30 days is shown in Fig. 18, which indicates barely no change for crystal form I as compared to that before the accelerated test (Fig. 6).

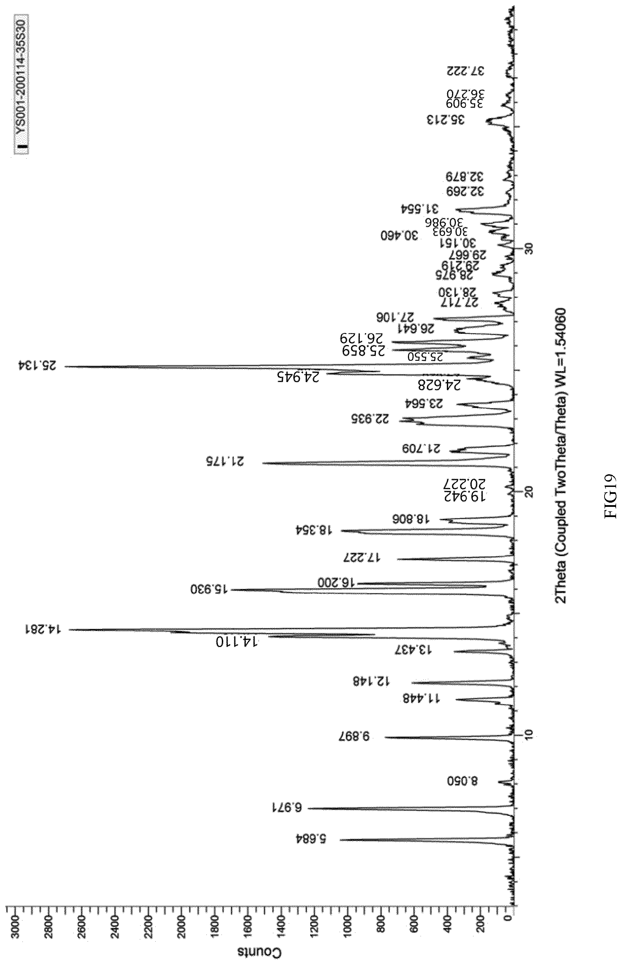

The XRD of crystal form II after the accelerated stability test for 30 days is shown in Fig. 19,which indicates barely no change for crystal form II as compared to that before the accelerated test (Fig. 7).

In summary, (R)-N-(5-(3-cyano-4-(3-methylmorpholinyl)quinolin-6-yl)-2-methoxypyridin-3-yl)methanesulfonamide in the literature (CN201610235304.5) was prepared as an amorphous form, which showed significant crystal transformation under high humidity test and high temperature test for 5 days, and thus was not suitable for development as a drug crystal form. In contrast, crystal forms I, II, III, IV and V of the present application all have obvious characteristic crystalline diffraction peaks, thus they can overcome the technical defect of amorphous form with no regular crystal structure. Crystal form I, crystal form II and crystal form IV of the present disclosure were stable under the conditions of high humidity test and high temperature test for 5 days, and crystal form I, crystal form II and crystal form IV were stable in accelerated test for 30 days. Therefore, the stability of crystal form I, crystal form II and crystal form IV of the present disclosure was significantly better than that of the amorphous form in the literature (CN 201610235304.5). Crystal form I and crystal form IV in the present disclosure can be used to prepare a variety of drugs. In addition, based on the solubility of crystal form I and crystal form IV in different environments and their stability within 24 hours, it is known that the drugs prepared by the polymorphic crystals in the present disclosure are easy to be absorbed by human body and possess excellent effects in the treatment of diseases.

Obviously, the above embodiments of the present disclosure are only examples to clearly illustrate the disclosure, and not to limit the implementation of the disclosure. The ordinary skilled person in the art, on the basis of the above description, can also make other different forms of changes or variations. It is not possible to exhaust all the embodiments herein, where all obvious variations or changes derived from the technical solutions of the present disclosure are still within the protection scope of the present disclosure.

## Claims

1. A crystal form of a fused ring compound having a structural formula of: 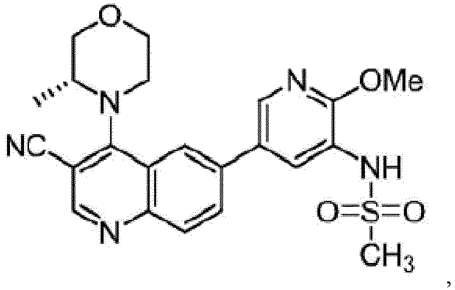 **characterized in that**, the crystal form is:
crystal form I, having characteristic diffraction peaks at 2θ angles of 11.3°, 17.2° and 21.1° using X-ray diffraction;
crystal form II, having characteristic diffraction peaks at 2θ angles of 25.1°, 21.2° and 14.1° using X-ray diffraction;
crystal form III, having characteristic diffraction peaks at 2θ angles of 6.6°, 13.4° and 8.0° using X-ray diffraction;
crystal form IV, having characteristic diffraction peaks at 2θ angles of 11.8°, 13.3° and 16.7° using X-ray diffraction; or
crystal form V, having characteristic diffraction peaks at 2θ angles of 6.5°, 13.3° and 20.0° using X-ray diffraction;
wherein the error in the 2θ angles is ±0.5°.

2. The crystal form according to claim 1, **characterized in that**,
crystal form I further has characteristic diffraction peaks at 2θ angles of 22.7°, 18.5°, and 13.8° using X-ray diffraction;
crystal form II further has characteristic diffraction peaks at 2θ angles of 16.0°, 7.0°, and 18.4° using X-ray diffraction;
crystal form III further has characteristic diffraction peaks at 2θ angles of 20.0°, 21.1°, and 10.5° using X-ray diffraction;
crystal form IV further has characteristic diffraction peaks at 2θ angles of 17.8°, 21.8°, and 24.4° using X-ray diffraction;
crystal form V further has characteristic diffraction peaks at 2θ angles of 10.4°, 24.0°, and 24.8° using X-ray diffraction;
wherein the error in the 2θ angles is ±0.5°.

3. A composition, **characterized in that** the composition comprises one of crystal form I, crystal form II, crystal form III, crystal form IV and crystal form V defined in claim 1 or 2, or any combination thereof, and crystal form I or crystal form IV accounts for 50% by weight or more of the composition.

4. The composition according to claim 3, **characterized in that** crystal form I or crystal form IV accounts for 80% by weight or more of the composition.

5. The composition according to claim 3, **characterized in that** crystal form I or crystal form IV accounts for 90% by weight or more of the composition.

6. A pharmaceutical composition comprising crystal form I, crystal form II, crystal form III, crystal form IV or crystal form V defined in claim 1 or 2, and a pharmaceutically acceptable carrier or excipient.

7. A method for preparing crystal form I defined in claim 1 or 2, **characterized in that** the method comprises adding a first organic solvent to the fused ring compound, heating to dissolve the fused ring compound, and adding a second organic solvent thereto, followed by stirring for crystallization, filtering and drying.

8. The method for preparing crystal form I according to claim 7, **characterized in that**, the first organic solvent is one of dichloromethane, trichloromethane and tetrahydrofuran, or any combination thereof, used in an amount of 3-5 times the weight of the fused ring compound, and the second organic solvent is one of acetone, butanone, ethanol, and ethyl acetate, or any combination thereof, used in an amount of 6-12 times the weight of the fused ring compound.

9. The method for preparing crystal form I according to claim 7 or 8, **characterized in that**, the stirring for crystallization is carried out at a temperature of 0-40°C for a time period of 4-15 hours, and the drying is carried out at a temperature of 60-150°C for a time period of 4-15 hours.

10. A method for preparing crystal form I defined in claim 1 or 2, **characterized in that**, the method comprises dissolving the fused ring compound in ethanol under heating, followed by stirring for crystallization, filtering and drying.

11. A method for preparing crystal form IV defined in claim 1 or 2, **characterized in that**, the method comprises dissolving the fused ring compound in an organic solvent and/or water under heating, followed by stirring for crystallization, filtering and drying.

12. The method for preparing crystal form IV according to claim 11, **characterized in that**, the method comprises dissolving the fused ring compound in a mixture of acetonitrile and water, wherein the amount of acetonitrile is 30-90 times the weight of the fused ring compound and the amount of water is 0-20 times the weight of the fused ring compound.

13. The method for preparing crystal form IV according to claim 11 or 12, **characterized in that**, the stirring for crystallization is carried out at a temperature of 0-40°C for a time period of 0.5-30 hours, and the drying is carried out at a temperature of 60-150°C for a time period of 1-20 hours.

14. Use of the crystal form according to claim 1 or 2 in manufacture of a medicament for inhibiting phosphatidylinositol 3-kinase.

15. The use according to claim 14, **characterized in that**, the medicament is an anti-tumor drug, and the tumor is selected from the group consisting of brain cancer, head and neck cancer, esophageal cancer, lung cancer, liver cancer, stomach cancer, kidney cancer, pancreatic cancer, prostate cancer, colorectal cancer, ovarian cancer, breast cancer, thyroid cancer, skin cancer, leukemia, myelodysplastic syndrome, sarcoma, osteosarcoma and rhabdomyosarcoma.

16. The use according to claim 14, **characterized in that**, the medicament is an anti-inflammatory drug or a drug for treatment of an autoimmune disease.

17. The use according to claim 16, **characterized in that**, the anti-inflammatory drug is a drug for treatment of chronic obstructive pulmonary disease or asthma, and the autoimmune disease is rheumatoid arthritis, psoriasis or systemic lupus erythematosus.
